# EUROPEAN PATENT APPLICATION

(11) **EP 3 996 060 A1**
(43) Date of publication of application: **11.05.2022**
(21) Application number: 21206051.1
(22) Date of filing: 02.11.2021
(51) Int. Cl.: G08B 17/113, G08B 1/08, G08B 17/10

(54) **SMOKE DETECTION SYSTEM MONITORING USING VISUAL CODE**

(30) Priority: 06.11.2020 US 202063198702 P
(71) Applicant: Carrier Corporation, Palm Beach Gardens, FL 33418 (US)
(72) Inventor: MUNUERA, Jose Manuel, 08950 Esplugues de Llobregat (ES); VIZUETE, Xavier, 08950 Esplugues de Llobregat (ES)
(74) Representative: Dehns

(57) **Abstract**

A system and method for monitoring conditions associated with a smoke detection system 100, including: operating the smoke detection system 100 to communicate data associated with at least one of the smoke detection system 100 and a protected area, from one or more detector modules 200, 202-206, to a display module 300, encoding the received data with the display module 300, generating a visual code 304 from the received data with the display module, displaying the visual code 304 in a display window 302 of the display module 200, 202-206, decoding the displayed visual code 304 with a mobile device 500 in wireless communication with the display module 200, 202-206, and displaying the decoded data on the mobile device 500 in a user-readable form.

Smoke detection system with detector modules and a visual module which are connected via backplane panels providing ease of installation. Information on the status of the smoke detection system is provided on the visual module in the form of a visual code (such as a barcode or a QR-code). The code is scanned by an authenticated mobile device which decodes it and presents the status information in a user-readable format.

## Description

The embodiments herein relate generally to smoke detection systems, and more particularly to improving fire detection system monitoring using a visual code, such as a matrix or quick response (QR) barcode ("QR code") to obtain data associated with the system and an associated protected area.

High Sensitivity Smoke Detector (HSSD) systems are typically positioned proximal to a protected area in a building and configured to monitor the protected area for smoke. These systems generally include an aspirating fan to draw air from a protected area via a network of sampling pipes and sampling holes. The sampled air is then passed through a high-sensitivity, precision detector (e.g., a detector module) that analyzes the air and generates a warning signal when appropriate (e.g., when smoke or combustion gases are present). Two or more of such systems may be linked together to share information, but each system is generally self-contained, requiring its own power source. The systems communicate with one another, and with other devices over a network and may be monitored by a user and/or an automated system.

Some HSSD systems also include a display module, linked to one or more detector modules. The display module may include a user interface such as a display window or a port (e.g., a USB port) to provide for local interaction between a user (e.g., an operator, installer, or the like), and the display module. Display windows are typically small, permitting only a limited system menu and/or a limited amount HSSD data to be displayed at a time. In instances where the user needs to extract a greater amount of HSSD data than can be displayed in the window at one time, such data can be obtained from a communication port (e.g., USB port), then uploaded and viewed on yet another device, such as a mobile computer. Having a USB port for extracting data associated with the system and the protected, area can add to manufacturing costs. In addition, it can be a less efficient way of obtaining data since it requires a user to have both a USB drive for extracting the data and a device for displaying the data.

What is needed then is a system and method for reducing HSSD system manufacturing costs, and increasing user efficiency by eliminating the need for a communication port for extracting data.

One embodiment provides a system for monitoring conditions associated with a smoke detection system, including: a first detector module including a sensor configured to detect smoke in a first volume of air received from a protected area, the first detector module electrically and physically coupled to a first backplane; a second detector module including a sensor configured to detect smoke in a second volume of air received from the protected area, the second detector module electrically and physically coupled to a second backplane and to the first backplane so as to transmit power, signals, or both between the first and second detector modules; a display module including a display window, the display module electrically and physically coupled to a third backplane, and to the first and second backplanes so as to transmit power, signals, or both between the display module and the first and second detector modules, the display module configured to: receive data from the first and second detector modules associated with at least one of the smoke detection system and the protected area, encode the received data, generate a visual code from the received data, and display the visual code in the display window; and a mobile device in wireless communication with the display module, configured to capture and decode the visual code, and display the decoded data on the mobile device in a user-readable form.

Optionally, the visual code is two dimensional.

Optionally, the two dimensional visual code includes a quick response code.

Optionally, the visual code is three dimensional.

Optionally, the three dimensional visual code includes a hologram.

Optionally, the mobile device includes at least one of a mobile phone, a personal digital assistant, a tablet, and a computer.

Optionally, the user-readable form comprises at least one of text, an image, a portable document format, table, a graph, a chart, a diagram.

Another embodiment provides a method for monitoring conditions associated with a smoke detection system, including: operating a smoke detection system to communicate data associated with at least one of the smoke detection system and a protected area, from one or more detector modules to a display module; encoding the received data with the display module, generating a visual code from the received data with the display module; displaying the visual code in the display window of the display module; decoding the displayed visual code with a mobile device in wireless communication with the display module; and displaying the decoded data on the mobile device in a user-readable form.

Optionally, the visual code is two dimensional.

Optionally, the two-dimensional visual code includes a quick response code.

Optionally, the visual code is three dimensional.

Optionally, the three dimensional visual code includes a hologram.

Optionally, the user-readable form includes at least one of text, an image, a portable document format, a table, a graph, a chart, a diagram.

Optionally, the method further includes selecting data for encoding from one or more display module menus.

Optionally, the mobile device comprises at least one of a mobile phone, a personal digital assistant, a tablet, and a computer.

The foregoing features and elements may be combined in various combinations without exclusivity, unless expressly indicated otherwise. These features and elements as well as the operation thereof will become more apparent in light of the following description and the accompanying drawings. It should be understood, however, that the following description and drawings are intended to be illustrative and explanatory in nature and non-limiting.

The following descriptions should not be considered limiting in any way. With reference to the accompanying drawings, like elements are numbered alike:
FIG. 1 illustrates a perspective view of a partially assembled smoke detection system, depicting a detector module being coupled with backplanes.
FIG. 2 illustrates a perspective view of the smoke detection system, once assembled.
FIG. 3 illustrates a schematic view of a smoke detection network.
FIG. 4 illustrates a block diagram of a mobile device.
FIG. 5A illustrates a screen shot of an exemplary display module first menu.
FIG. 5B illustrates a screen shot of an exemplary display module second menu showing a user choosing to view an event log.
FIG. 5C illustrates a screen shot of an exemplary display module third menu prompting a user to select the data to be encoded in a visual code.
FIG. 5D illustrates a display module screen shot of a generated visual code.
FIG. 6A illustrates a mobile display showing a scanned visual code and displaying smoke detection system data.
FIG. 6B illustrates another mobile display, displaying smoke detection system data.
FIG. 7A illustrates a screen shot of an exemplary display module fourth menu prompting a user to select the data to be encoded in a visual code.
FIG. 7B illustrates another mobile display displaying smoke detection system data.
FIG. 8 illustrates a method for monitoring conditions associated with a smoke detection system.

A detailed description of one or more embodiments of the disclosed apparatus and method are presented herein by way of exemplification and not limitation with reference to the Figures. Reference will now be made in detail to various embodiments of the present teachings, an example of which is illustrated in the accompanying drawings. Wherever possible, the same reference numbers will be used throughout the drawings to refer to the same or like parts.

In the following description, reference is made to the accompanying drawings that form a part thereof, and in which is shown by way of illustration specific implementations in which may be practiced. These implementations are described in sufficient detail to enable those skilled in the art to practice these implementations and it is to be understood that other implementations may be utilized and that changes may be made without departing from the scope of the present teachings. The following description is, therefore, merely exemplary.

In general, HSSD systems, such as smoke detection system 100 and smoke detection network 400 may include one or more detector modules 200-206 that sample air from a protected area via a network of sampling pipes and sampling holes. Such HSSD systems may also include a display module 300 that, among other things, permits a user to obtain information associated with the HSSD system and the protected area. The display module 300 may be configured to generate a visual code 304 that includes real-time or near real-time encoded data associated with the HSSD system and/or the protected area, as described below. The visual code 304 may then be viewed on a display window 302 of the display module 300. The generated visual code 304 may then be captured and decoded (e.g., scanned and read) by an authenticated mobile device 500 (e.g., a mobile device that is authorized to read the visual code).

The mobile device 500 may include a mobile phone (smart phone), personal digital assistant (PDA), tablet computer, mini-tablet, laptop computer, mini-laptop, and/or other smart device that provides continuous or periodic sensing and combined sensor data from a combination of sensors, such as motion, biometrics, and/or GPS (e.g., a smart watch or fit device), etc., and may include a device that has or may only have short range or local-area wireless capabilities. Mobile phone generally refers to a mobile handset with at least cellular telephonic communications capabilities, computing capabilities, and wireless/short-range data communication capabilities (e.g., Bluetooth, RFID, NFC, etc.) and may also include GPS capabilities. The mobile device 500 may be configured to have a mobile application 572 that permits a user to capture and decode the visual code 304, and display such decoded information on the mobile device 500 in user-readable form.

In general, the term "visual code" may be defined as a symbolic and/or geometric representation of information that can be deciphered from such representation. In general, a visual code 304 may be compactly represented by a series of bars or a graphical symbol such as a two dimensional barcode or a QR code, or may be represented by an image, such as a three dimensional hologram. While the disclosure refers generally to a visual code 304, which may be a two dimensional representation of information such as a QR code, it is understood that the visual code 304 may also include three dimensional images.

A QR code is a two-dimensional barcode that use dark and light modules arranged in a square configuration to encode data for being optically captured and decoded by a machine (e.g., a mobile device). Some QR codes also utilize color or images as part of the QR code. Elements of the barcode define the version, format, position, alignment, timing, etc., to enable capturing and decoding. (The particular details of detecting, capturing and decoding a QR code are known in the art and are not detailed here). The remainder of the barcode can encode various types of information, in any suitable format, such as binary, alphanumeric, etc. and the barcode can be based on any of a number of standards.

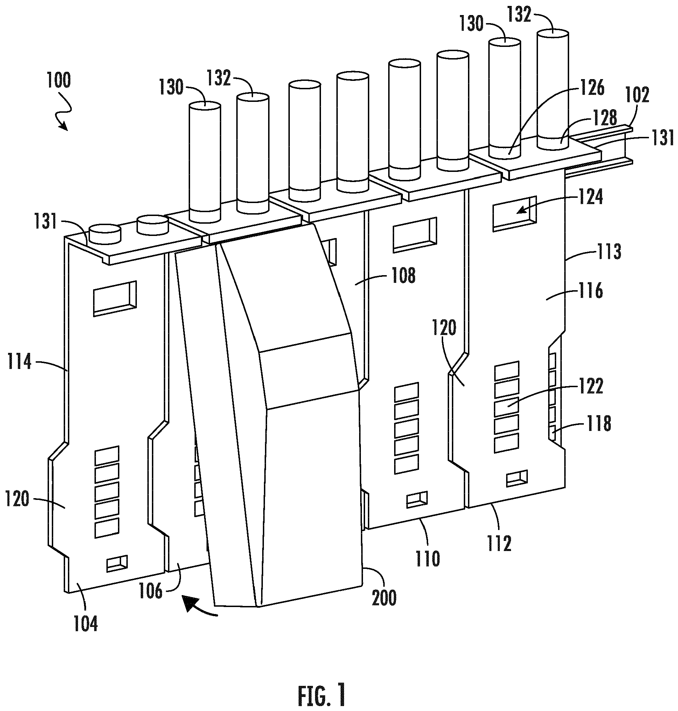

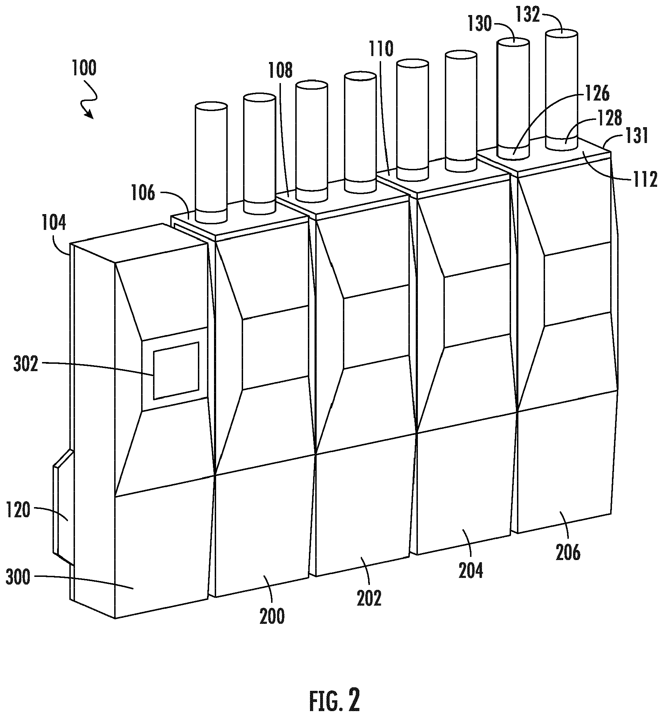

FIG. 1 illustrates a perspective view of a partially assembled smoke detection system 100 with a detector module 200 being coupled with a backplane 106 and FIG. 2 illustrates the smoke detection system 100 after coupling the detector module 200, a plurality of additional detector modules 202-206, as well as a display module 300, to backplanes 104-112.

The smoke detection system 100 may be modular, scalable, and configured for rapid installation during construction of a new facility or for a retrofit in a pre-existing facility. The smoke detection system 100 may be a "multichannel" system, being configured to receive and analyze inputs from multiple air sources and determine the presence of smoke, combustion gasses (e.g., carbon monoxide), elements or compounds indicative of smoke and/or fire, or other harmful elements or compounds in the air received. Accordingly, one multichannel system 100 may be configured to monitor multiple protected areas, while using a single power source, for example. Further, such multichannel smoke detection systems 100 may be linked together and/or with other monitoring devices via any suitable telecommunications network, e.g., existing open systems interconnection network protocols including but not limited to Layer 1 protocols such as RS-485 or Ethernet, and/or one or more wireless protocols, and may be linked to an external building management system 426 which may include a fire panel, enabling or otherwise facilitating centralized monitoring. Further, the smoke detection system 100 may be configured to provide instructions to the building management system 426, so as to promote a rapid response to any emergency situation.

In general, the smoke detection system 100 includes a rail 102 and one or more backplanes (e.g., five shown 104-112 in FIG. 1). The backplanes 104-112 and the rail 102 may be coupled, for example, hung, fastened, mounted, or otherwise attached to a wall, so as to be suspended therefrom. The backplanes 104-112 may be directly fastened to the wall using screws. The rail 102 may serve to facilitate installation and/or accurate alignment of the backplanes 104-112 in a rack, such as a metallic rack. Clips may be employed to initially secure the backplanes 104-112 to the rail 102, stabilizing the backplanes 104-112 while the backplanes 104-112 are fastened to the wall. In some installations, the rail 102 may be unnecessary and omitted. Although five backplanes 104-112 are illustrated in Figure 1, it will be appreciated that fewer backplanes may be used, or additional backplanes may be employed, for example, to provide for fewer or add additional channels to the smoke detection system 100, respectively, as will be described below. Further, in examples including the rail 102, the rail 102 may be sized to accommodate any suitable number of backplanes 104-112.

Each of the backplanes 104-112 may include first and second lateral sides 113, 114 and an outward face 116 extending laterally between the first and second lateral sides 113, 114 and facing away from the rail 102, for example. Further, each of the backplanes 104-112 may include an interconnection port 118 formed on the first lateral side 113, for example, recessed or otherwise offset from the outward face 116. The backplanes 104-112 may also each include an interconnection terminal 120 protruding from the second lateral side 114. The interconnection port 118 and the interconnection terminal 120 may be complementarily shaped, such that the interconnection terminal 120 of each of the backplanes 104-112 is configured to be mate with the interconnection port 118 of an adjacent one of the backplanes 104-112. It will be appreciated, however, that other arrangements may be contemplated for the interconnection port 118 and interconnection terminal 120. For example, the interconnection terminal 120 may be provided by a flat cable or plug, which is received into one or more recessed connections or sockets, provided by the interconnection port 118.

Each interconnection port 118 and each interconnection terminal 120 may be or include one or more electrical contacts. The electrical contacts may be provided for transferring power and/or one or more signals between the backplanes 104-112, enabling communication and/or power transfer between the backplanes 104-112 and, more particularly, the modules associated therewith, as will be described in greater detail below. Such communication signals may follow various network protocols, whether the same or different between the two communication links.

Each of the backplanes 104-112 may also include a module port 122, for example, on the outward face 116. The module port 122 may be disposed on the bottom half of the backplane 104-112; however, such positioning is merely one example among many contemplated herein. Further, the module port 122 may include one or more electrical contacts configured to transfer power between and/or communicate with one or more modules coupled with the backplanes 104-112, as will be described in greater detail below. The electrical contacts of the module port 122 may be electrically coupled with the interconnection port 118 and/or the interconnection terminal 120, via the outward face 116 of the backplane 104-112, so as to provide electrical transmission therebetween. Further, the illustrated module port 122 may be representative of any arrangement of one or more electrical contacts, connections, and/or interfaces provided by the backplanes 104-112 for communication with and/or powering associated modules, as will be described below. The backplanes 104 and 112 positioned at the ends of the provided series of backplanes 104-112 may have an open interconnection port 118 and/or an open interconnection terminal 120. For example, as shown, the backplane 104 may have an open interconnection terminal 120, while the backplane 112 may have an open interconnection port 118, thereby facilitating expansion of the system 100.

The backplanes 104-112 may be modular and interchangeable; thus, it will be appreciated that the detector modules 200-206 and the display module 300 may be disposed in any suitable order and to any of the backplanes 104-112, such that any of the backplanes 104-112 are disposed adjacent the first or second lateral side 113, 114 of any of the other backplanes 104-114. Moreover, the number of backplanes 104-112 may exceed the number of detector modules 200-206 by at least one, to provide the backplane 104 for the display module 300. The backplane 104-112 for receiving the display module 300 may be chosen, for example, according to the location of the conduits 130, 132, such that the backplane 104-112 for the display module 300 may not be aligned with the conduits 130, 132.

Since coupling with the conduits 130, 132 may not be required for the backplane 104 that couples with the display module 300, the top side wall 131 of the backplane 104 may be removed or omitted, as shown. In another non-limiting example, the top side wall 131 may be retained and not employed or employed for any other reason, such as to increase retention of the display module 200. Further, the system 100 may be configured to operate with one or more of the detector modules 200-206 removed, leaving one or more of the backplanes 104-112 empty but still coupled to the rail 102 and to one or more adjacent backplane(s) 104-112.

It will be appreciated that the detector modules 200-206 and/or display module(s) 300 and/or backplanes 104-112 may be combined. Further, detector modules 200-206 can be differently oriented. For example, the detector modules 200-206 may be oriented as illustrated in FIG. 2. In another non-limiting example, one or more detector modules (e.g., 200, 204), may be rotated 180° (not shown) relative to the position of the display module 300. Each of the backplanes 104-112 may also include one or more module hangers 124. The module hanger 124 may be a bracket, clip or set of clips, an aperture exposing a portion of the rail 102, a secondary rail, a recess or ledge, or a protrusion extending generally normal to the outward face 116. A variety of configurations for the module hanger 124 are contemplated for use herein.

The display module 300 may also include a mounting member, configured to couple with the module hanger 124 of one of the backplanes 104-112, and/or the rail 102, so as to secure, hang, mount, or otherwise physically couple the display module 300 and the backplane 104. It will be appreciated that the module hanger 124 and the mounting member may be a hook and ledge pairing, one or more magnets, a collet, interlocking members, a dovetail connection, or any other suitable connection or coupling, any of which is contemplated by use of the term "physically couple," as used herein.

Each of the backplanes 104-112 may also include an outlet port 126 and an inlet port 128. The outlet and inlet ports 126, 128 may each be coupled to a conduit 130, 132, with the outlet port 126 coupling with the conduit 130 and the inlet port 128 coupling with the conduit 132, as shown. The conduits 130, 132 may be air conduits of any type and may couple with the outlet and inlet ports 126, 128, for example, by sliding into the ports 126, 128 using slip couplings, press-fitting, fasteners, gaskets, seals, adapters, and/or the like. The conduit 132, coupled to the inlet port 128, may extend from and one or more protected areas and may receive air therefrom and transmit the air to the inlet port 128. The conduit 130, coupled to the outlet port 126, may receive air from the outlet port 126, and transmit the air to any suitable location, for example, back to the one or more protected areas, to the exterior environment, or to any other area.

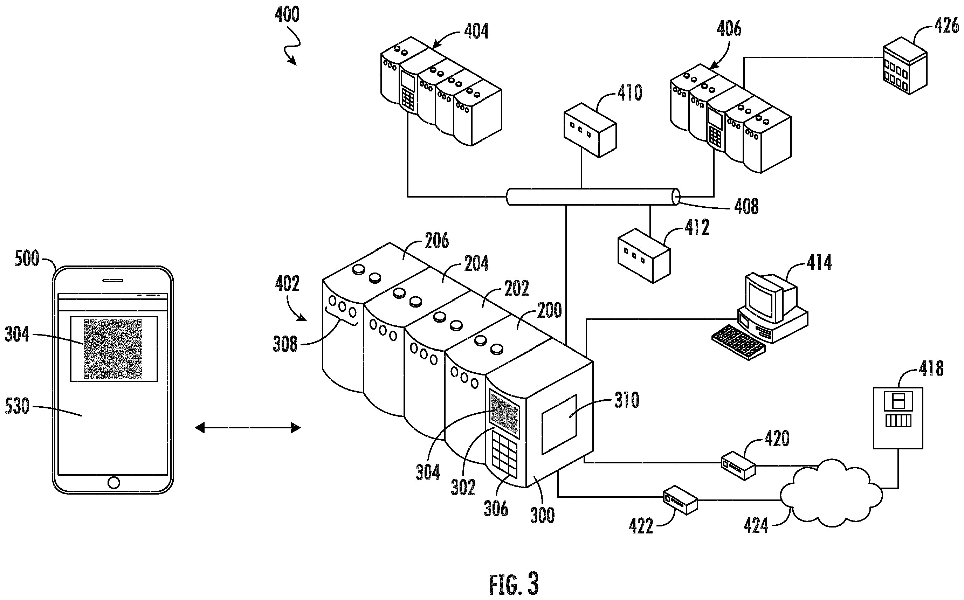

Turning now to FIGS. 2 and FIG. 3. FIG 3 illustrates a schematic view of a smoke detection network 400. Each of the detector modules 200-206 may be coupled with one of the inlet air conduits 132 via one of the inlet ports 128 and with one of the outlet air conduits 130 via one of the outlet ports 126. Further, the detector modules 200-206 may each include an aspirating fan or blower, housed internally therein, which may be configured to draw air into the detector module 200-206 and exhaust air out of the detector module 200-206 via the air conduits 132, 130, respectively. The detector modules 200-206 may each also include a dust filter to remove particulate matter from the air drawn into the detector module 200-206 and an air flow sensor to monitor the flow rate of the air. Additionally, the detector modules 200-206 may each include a sensing device, such as a laser head or another type of high-sensitivity smoke detection device, configured to sense elements, compounds indicative of smoke in the air, and/or combustion gasses such as carbon monoxide, and provide signals indicative of the same. The sensing device may include a filter to remove particulate matter over a certain size. The detector modules 200-206 may each include a controller and memory to receive, interpret, and/or record such signals.

Each of the detector modules 200-206 may provide one or more interfaces, for example, an internal interface, an external interface and a user interface, each of which may be configured to provide access to the controller and/or provide access for obtaining and/or extracting data associated with the system and/or the protected area, each as further described below.

The detector modules 200-206 may include a plurality of LEDs 308, configured to indicate power, alarm, fault, and disable. Further, the detector modules 200-206 may include two inputs providing supervised voltage detectors and three outputs providing free voltages relays for communications with an external device, such as a fire panel and/or with a building management system 426. The inputs of the detector modules 200-206 providing supervised voltage detectors may enable the inputs to recognize a status of another device coupled to the detector module 200-206 therewith. Thus, the input including a supervised voltage detector may be configured to recognize that: a signal is present (input activated), no signal is present (input not activated), an open circuit is exists (broken line between the fire panel and the input), and a short circuit exists (short-circuited line between the fire panel and a detector).

The detector modules 200-206 may also provide a network protocol for communicating the display module 300 and/or external devices (e.g., a fire panel) and/or a building management system FIG. 3, 426, and may include an external power supply port, for example, configured to receive 24V power from the external power source. These interfaces may all be configured to be integrated with the backplanes 104-112. Further, the backplane 104-112 may also provide connectivity between the display module 300 and an analogue loop for connecting any existing fire panel network protocol.

A detector module 200-206 and/or the display module 300 may include a communication interface which may include one or more devices or modules configured to communicate via point-to-point (e.g., device pairing), one-to-many (e.g., broadcasting) or mesh network, such as a Wi-Fi and/or a Bluetooth^{®} low energy communications (BLE) module and/or near-field communication (NFC) devices, NFC-enabled devices, NFC-equipped devices or ultra-wide band (UWB) enabled devices. The communication interface may employ one or more protocols for network communication including, but not limited to: NB-IoT, eMTC, EC-GSM-IoT, LTE-M, DASH7, NB-FI, LPWAN, Ethernet, SAP, SAS, ATP, Bluetooth, GSM, TCP/IP, WiFi, ZigBee, 6LoWPAN, CAT6 Ethernet, HomePlug, and NFC communication interfaces and protocols, including NFCIP-1, NFCIP-2 and SNEP. Such communication interface may permit communication between display module 300 and one or more detector modules 200-206, and/or among detector modules. In addition, the communication interface may also permit communication between a mobile device 500 having a mobile device display 530 ("mobile display") and at least one of a detector module 200-206 and a display module 300, as further described below.

Since the detector modules 200-206 provide a network protocol then the smoke detection system 100 may omit the display module 300. The detector modules 200-206 may operate in a "standalone" mode, providing a direct communication between the detector modules 200-206 and an external device. As such, the detector modules 200-206 may be configured to individually or collectively report signals to a remote fire panel or to other external devices or systems, such as a building management system (BMS) 426.

The detector modules 200-206 and/or the display module 300 may be configured to communicate with external HSSD systems (whether other smoke detection systems 100 or conventional HSSD devices) via an external interface integrated with the backplanes 104-112. Such external interface may provide for integration of the detector modules 200-206 into a larger system or network, enabling the controller to communicate with external hardware and/or users, for example, obviating a necessity for an intermediary such as the display module 300. The internal interface may provide for communication between the controllers of the detector modules 200-206 and the display module 300.

The smoke detection system 100 may further define a protocol for communication between the display module 300 and the detector modules 200-206, which may be integrated in the backplanes 104-112. This protocol may be based on a token ring topology without a master. Further, each detector module 200-206 and/or each display module 300 may include at least two inputs: a programmable input disposed proximal the top and a second programmable input for power supply unit (PSU) monitoring. In some cases, PSU monitoring may not be required and, as such, the second programmable input could be programmed for other purposes. The detector modules 200-206 and/or display modules 300 may also include outputs. Each output may be activated by a volt free relay contact placed in the backplanes 104-112, e.g., for optimizing the number of pins in the MCU connector. The switching contact masy use the power supply present in the interconnect board, that is from 18 VDC to 30 VDC.

The display module 300 may include a user interface, an external interface, an internal interface, a controller, and one or more memory devices associated therewith. The user interface may provide for local interaction between an operator, installer, or the like, with the display module 300. The external interface may provide for integration of the display module 300, and thus the system 100, into a larger system or network, as will be described in greater detail below. The controller and memory devices may process, store, and/or transmit information via any of the interfaces and/or may be employed to control, calibrate, or otherwise configure other components of the system 100. The display module 300 may not require connection with an air conduit and thus the inlet and outlet ports 128, 126 of the backplane 104 coupled with the display module 300 may not be connected with any air conduits.

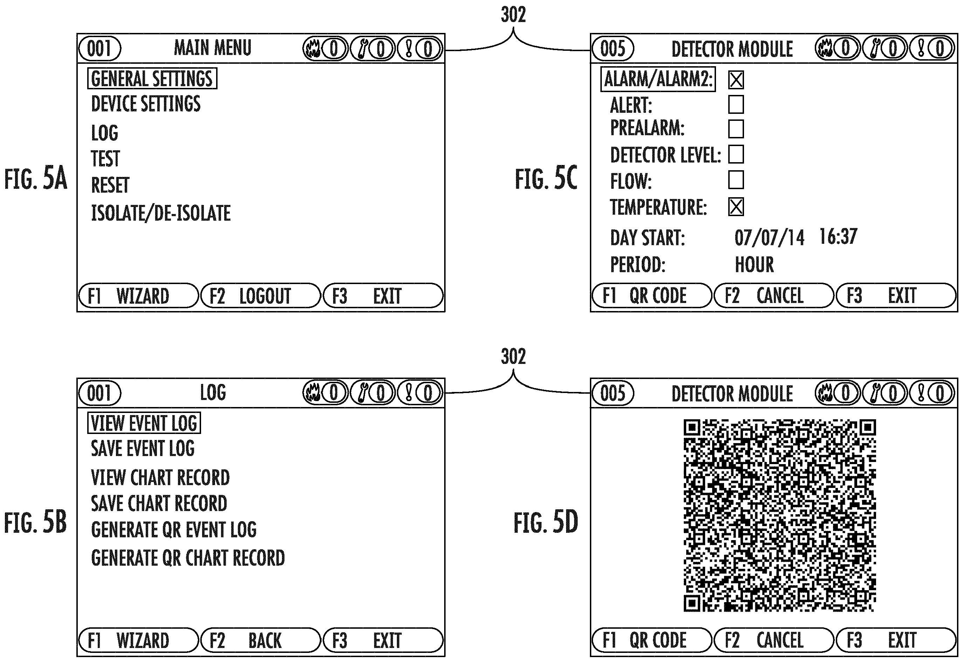

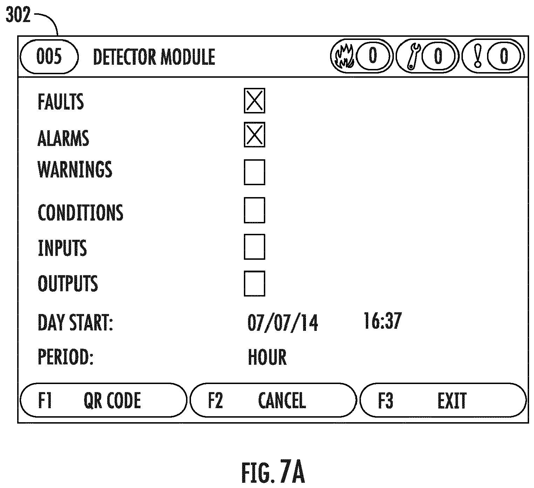

The display module 300 may be configured to have pre-programmed menus that are accessible by a user via a user interface such as a display window 302 and/or a keypad 306. The pre-programmed menus may allow a user to configure one or more detector modules 200-206, display modules 300, the smoke detection system 100 and/or the smoke detection network 400. The display module 300 may be configured: to have pre-programmed menus FIG. 5A-5D, 7A that permit a user to select the type of data to be encoded in a visual code 304, such as a QR code; and to generate and display the encoded visual code 304 in the display window 302, as further described below.

Display module 300 may include a visual code generator module 310, configured to generate a visual code 304 (e.g., a QR code) for display on a display window 302. The visual code 304 may include real-time or near real time data and/or historical data associated with one or more of the smoke detection system 100, the smoke detection network 400, detector modules 200-206, and a display module 300. Examples of visual code data may include event tracking, such as triggered alarms, triggered alerts, alarms, pre-alarms, faults, warnings, detector level, airflow rate, settings, inputs, outputs, temperature, date, time, and location. The data is displayed in a visual code (e.g., QR code) format that is readable by an authorized (e.g., authenticated) mobile device 500, as further described below.

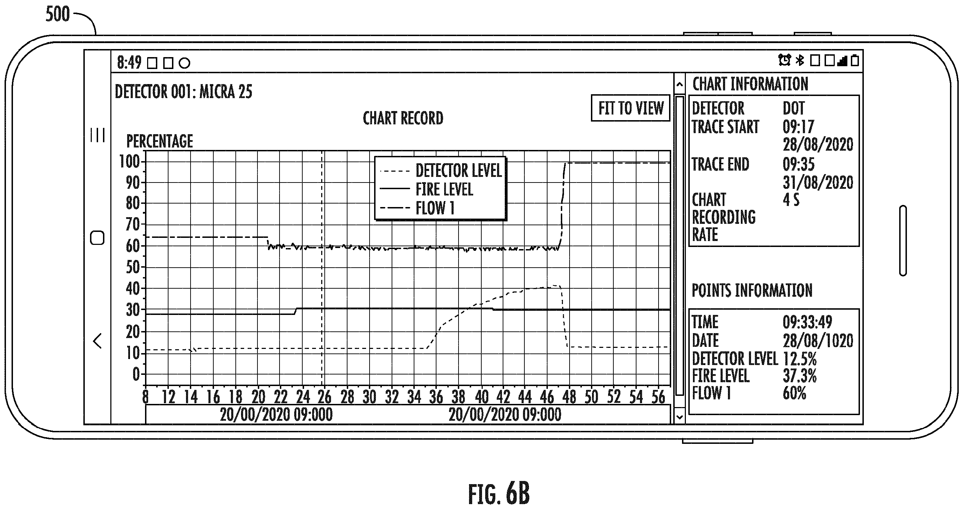

The display module 300 may be configured with a learning mechanism that may be in one-way or two-way communication with the visual code generator module 310. The learning mechanism may be a program utilized to obtain real-time or near real-time data from at least one of the smoke detection network 400, one or more detector modules 200-206, and the protected area. The detector module 300 may then be configured to present the learning mechanism data to the user in a visual code 304. For example, the learning mechanism data and/or prior visual code data may be stored in a memory (e.g., volatile and/or non-volatile). The display module 300 may be configured such that the learning mechanism data may be compared against previously generated visual code data. The display module 300 may be configured to generate an updated or modified visual code 304 based on the such comparison. It can be appreciated that the presentation of the newly encoded data may take various forms. By way of example, when scanned by mobile device 500, the decoded data may be presented as a "chart record" as shown in FIG 6B, which may compare "airflow" real-time or near real-time data, to historical data (e.g., when a visual code as previously generated). The display module 300 may be configured to trigger the learning mechanism to obtain data and generate a visual code 304 (or an updated or modified visual code based on a previously generated visual code), for example, when an authorized mobile device 500 is in proximity of the display module 300. The display module 300 may generate a visual code allowing user may obtain information without having to interact with one or more display module menus.

Referring now generally to Figures 1-3, in exemplary operation, each of the detector modules 200-206 may receive and monitor air, and thus each may provide a separate "channel" in the system 100. For example, each detector module 200-206 of the system 100 may be configured to receive and monitor air from separate protected areas. However, in some instances, multiple detector modules 200-206 or "channels" may be allocated to a single protected area, for example, to provide redundancy in case one of the detector modules 200-206 fails. Further each detector module 200-206 may provide a closed portion of an air flowpath, such that leakage or "crosstalk" between the flowpaths of the detector modules 200-206 is avoided and/or eliminated.

The number of channels desired may be pre-selected. Once the desired number of channels is determined, a number of backplanes 104-112 may be selected, which may generally be at least one more than the number of channels. A number of detector modules 200-206 may also be selected, which may correspond to the number of channels desired. It will be appreciated that the number of detector modules 200-206 "corresponding" to the number of channels does not necessarily require a 1:1 relationship, as two or more detector modules 200-206 may be provided for a single channel and/or two or more channels may be monitored by a single detector module 200-206. Moreover, when different types of detector modules 200-206 are employed for the first and second orientations, the number of each type of detector modules 200-206 may also be determined.

The display module 300 may receive instructions from a user, an external system, or the like, which may be used to configure the detector modules 200-206, passing such signals between the display module 300 and the detector modules 200-206 via the backplanes 104-112. When on-line, the detector modules 200-206 may monitor air from the protected areas, record events, and/or provide signals indicative of such monitoring (e.g., upsets, alarms, etc.) to the display monitor 200 and/or to an external device, such as a fire panel. The display module 300 may be coupled with a detector management device, such as a building management system 426, a graphical user interface, an external computer or controller, a master controller, a screen, another device, and/or a combination thereof and may relay the monitoring information to this detector management system.

The smoke detection network 400 may include three or more module "clusters" (three are shown: 402, 404, 406). Each cluster 402, 404, 406 may be provided by one or more of the system 100 shown in and described above with reference to Figures 1-2, and thus may include the display module 300 and one or more of the detector modules 200-206, as indicated for cluster 402. Each of the clusters 402, 404, 406 may be configured to receive and monitor air from separate protected areas, and may each include any number of detector modules 200-206.

The clusters 402, 404, 406 may be linked together via a network 408, which may include one or more computers, servers, junctions, or the like, and may be, for example, an Ethernet or one or more wireless links. The network 408 may be a closed network or may include, for example, a secured connection via the internet. One or more, for example, as shown, two conventional smoke detectors 410, 412 may also be linked to the network 408. The conventional smoke detectors 410, 412 may or may not be classified as "high-sensitivity" smoke detectors, as the term is known in the art, and may be configured to receive and monitor air from secondary protected areas, for example, if the clusters 402, 404, 406 are introduced to the system 400 as a retrofit. The conventional smoke detectors 410, 412 may be used as a back-up or redundant monitoring, in addition to the clusters 402, 404, 406.

Optionally, at least one display module 300 of one, some or all of the clusters 404, 404, 406, may include a user interface that accepts input from a user. Examples include a keyboard, a mouse, microphone (e.g., for capturing voice commands), and/or one or more of a display window 302 and keypad 306. The user may be able to access the cluster 402, for example, the controller of the display module 300, using the controller host 414. The display module 300 may be coupled with a fire panel 418, for example, via an analogue protocol interface card (APIC) such as an encoder 420 or decoders 422, and one or more analog loops 424. It will be appreciated that the display module 300 may only communicate with one APIC at a time, and thus the depicted connection with the encoder 420 and decoder 422 may represent the ability to connect to either the encoder 420 or the decoder 422. Further, there may be six or seven different loop protocols corresponding to the different of fire panels and provided as part of the analogue loop 424.

The clusters 402, 404, 406 may be controlled in a token ring network topology. Alternatively, the clusters 402, 404, 406 may be controlled in any other network topology, e.g., in one or more "master-slave" relationships. Although such master-slave topology may be slower than a token ring network topology, such a master-slave topology may allow for backwards compatibility with the conventional smoke detectors 410, 412. For example, the slave clusters 402, 404 and/or the convention smoke detectors 410, 412 may report to the master cluster 406. The master cluster 406 may, in turn, report to an overall building management system 426, for example, via a building management system protocol, or in some other cases, via for example, Ethernet connection or other Layer 1 network protocols. Further, the display modules 300 may be able to control and/or monitor a variety of systems, including the building management system, for example, mechanical and/or electrical equipment such as ventilation systems, lighting and/or power systems, security systems, fire systems, e.g., as part of the building management system, and the like. Various protocols may be supported, including but not limited to MODbus. The master cluster 406 may monitor the building management system 426 and/or provide instructions thereto.

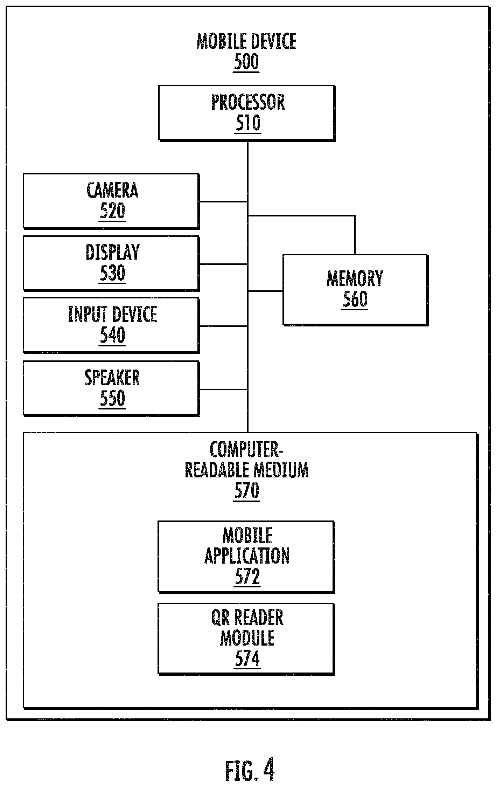

Turning to FIG. 4, with further reference to FIG. 3, shows a block diagram of a mobile device 500. The mobile device 500 includes a processor 510, a camera 520, a mobile display 530, an input device 540, a speaker 550, a memory 560, and a computer-readable medium 570.

Processor 510 may be any suitable processor operable to carry out instructions on the mobile device 500. The processor 510 is coupled to other units of the mobile device 500 including camera 520, display 530, input device 540, speaker 550, memory 560, and computer-readable medium 570.

Camera 520 may be configured to capture one or more images via a lens located on the body of the mobile device 500. The captured images may be still images or video images. The camera 520 may include a CMOS image sensor to capture the images. Various applications (e.g., mobile application 572) running on processor 510 may have access to camera 520 to capture images. It can be appreciated that camera 520 can continuously capture images without the images actually being stored within the mobile device 500. Captured images may also be referred to as frame images. The camera 520 may be configured to capture images of a machine readable code, e.g., a visual code 304, such as a QR code.

Mobile display 530 may be any device that displays information to a user. Examples include an LCD screen, CRT monitor, or a touch screen.

Input device 540 may be any device that accepts input from a user. Examples include a touch screen, keyboard, keypad, mouse, or microphone, and include virtual equivalents of the same (e.g., virtual keyboard, etc.). In the case of a microphone, the microphone may be any device that converts sound to an electric signal.

Speaker 550 may be any device that outputs sound to a user. Examples may include a built-in speaker or any other device that produces sound in response to an electrical audio signal. Speaker 550 may be used to provide a voice sample for purposes of authentication, or launch a mobile application (mobile app) for interfacing with the display module 300.

Memory 560 may be any magnetic, electronic, or optical memory. It can be appreciated that memory 560 may include any number of memory modules. An example of memory 560 may be dynamic random access memory (DRAM).

Computer-readable medium 570 may be any magnetic, electronic, optical, or other computer-readable storage medium. Computer-readable storage medium 550 may include any combination of volatile and/or non-volatile memory such as, for example, buffer memory, RAM, DRAM, ROM, flash or any other suitable memory device, alone or in combination with other data storage.

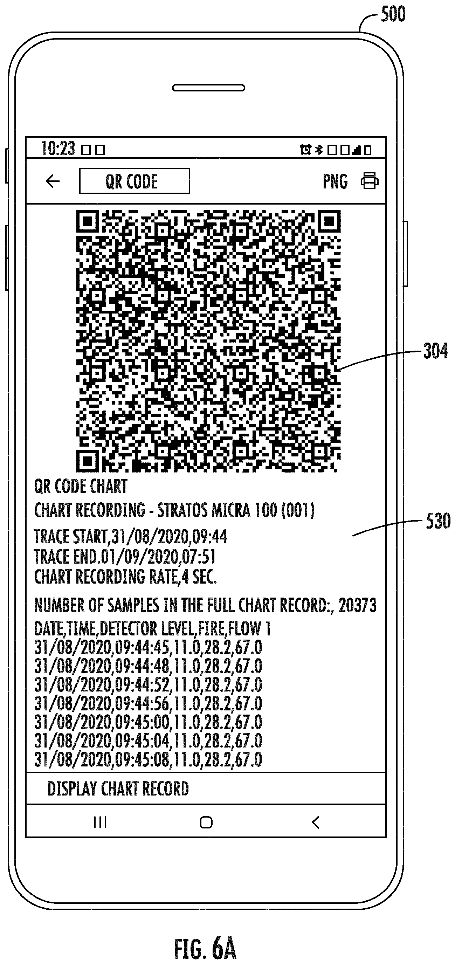

Mobile application 572 can be any application executable by a processor on the mobile device 550. For example, the mobile application may be a secure application for authenticating a user permitted to access the smoke detection system 100, including the display module 300. An authorized user may be permitted, among other things, to access menus of the display module 300. See, FIGS. 5A-D, 7A. The mobile application 572 may be configured to capture the visual code 304, decode the encoded data, and display the decoded data in a form that is understandable to the user. By way of example, and not limitation, the decoded data may be in the form of text as illustrated in FIG. 6A and 7A, a text file (e.g., comma-separated values), a portable document format (PDF), a graphical file (e.g., TIFF, GIF, JPG, etc.), a spreadsheet, a table, graph, or chart (e.g., FIG. 6B), and/or a diagram.

A code reader module 574 may be configured to capture and decode a visual code 304. The code reader module 574 may interface with camera 520 to capture a visual code generated by display module 300. Upon capturing the visual code 304, the code reader module 574 can decode the visual code 304 and convert it to useful information, such as an event log and/or a chart record as further described below.

Turning to FIGs. 5A-D, and 7A, with reference to FIGs. 1-4, screen shots of one exemplary series of menus associated with application functions of a display module 300, are shown by way of example. It should be appreciated that menus and associated application functions may vary. Each of FIG. 5A-D and FIG. 7A illustrate an active ("on") display window 302. The display window 302 may be in an active "on" state while the smoke detection system 100 is operational. The display window 302 may be deactivated (e.g., in "sleep mode") until activated by a user through an interaction. For example, the display window 302 may be activated when the user interacts with display module 300 (e.g., touching the display screen). In yet another example, the display window 302 may be activated when the user and/or the mobile device 500 has been authenticated by the smoke detection system 100. For example, the user and/or the mobile device 500 has been authenticated by at least one of the smoke detection network 400, the smoke detection system 100, and a display module 300. In yet another non-limiting example, the display window 302 may be activated when an authenticated mobile device 500 comes within proximity of the display module 300, via a wireless data exchange between the mobile device 500 and the display module 300.

FIG. 5A illustrates a screen shot of an exemplary display module first menu. The display module 300 may be configured such that when a user interacts with the display window 302, a first menu (e.g., "main menu") may allow a user to select the type of information to be encoded in a visual code 304, such as a QR code. From the main menu in FIG. 5A, a user may select "log" referring to a log of events associated with at least one of the smoke detection system 100, the smoke detection network 400, a detection module 200-206 and a display module 300. By selecting "log", the user may be presented a second menu, which may allow a user to select whether to view or save an event log, view or save a chart record, and/or generate an event log or chart record. In this example, a user may select "View event log" as illustrated in FIG. 5B. A user selecting "View chart record" may be presented a different menu as illustrated in FIG. 7A, discussed below.

The display module 300 may be configured to allow a user to select one or more types of data or data parameters as illustrated in FIG. 5C. For example, a user may desire information about the protected area, such as whether any alarms, alerts, or prealarms have been triggered or are presently active. Other information may relate to temperature, or airflow throughout the smoke detection network 400. For example, information about airflow may indicate whether a detector module 200-206 requires service, or a sampling hole or sampling pipe may be obstructed. A user may select the information by date and/or time, or over a range of dates/times. Other information may relate directly to the smoke detection system 100 or smoke detection network 400, such as faults, alarms, warnings, conditions, inputs and outputs, as illustrated in another exemplary menu as shown in FIG. 7A.

The display module 300 may be configured to permit the user to return to the prior menu (FIG. 5B) and make another selection. For example, a user may desire to "Generate QR event log" to generate a QR code as illustrated in FIG. 5D. The generated visual code 304 represents the data selected from the menu illustrated in FIG. 5C. Depending on user selection or preferences, the encoded data may be real-time, near real-time or historical data. It is understood that selecting a similar request to generate a visual code (e.g., "Generate QR chart record") would encode the data selected from an associated menu. It is further understood that a menu pay permit a user to generate a visual code such as a hologram or other three dimensional code.

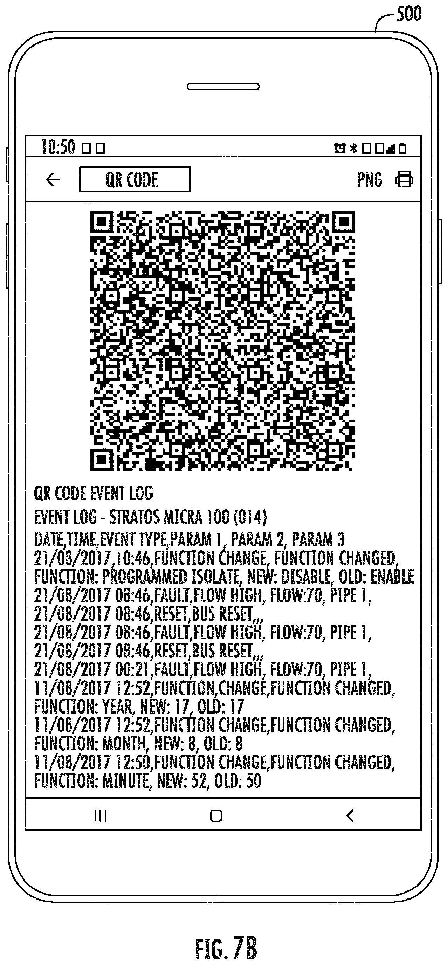

Turning to FIG. 6A-B and FIG. 7B, a mobile display 530 is shown. Once a visual code 304 is generated by the display module 300, it may be captured (e.g., read or scanned) using the mobile device camera 520 which may be accessible through the mobile application 572 (e.g., via the code reader module 574). The mobile application 572 may display the scanned visual code 304 as shown in FIG. 6A. The mobile application 572 may display data associated with the visual code in a text format (FIG. 6A, 7B) and/or in a readable chart format (FIG. 6B). The mobile application 527 may be configured to permit the user to scroll through the data, or screen capture the data which may be transmitted wirelessly (e.g., as a PDF, JPG, TIFF, etc.) to another mobile device 500, to a network or cloud based server, or to an external device (e.g., fire panel, building management server, printing device, etc.). FIG. 7B is similar to FIG. 6A, in all material respects, except that FIG. 7B illustrates the visual code event log data in a text format.

With additional reference to FIGs. 1-7, FIG. 8 illustrates a flowchart of an exemplary method 800 for monitoring conditions associated with a smoke detection system 100. The exemplary method 800 begins at 802 with operating a smoke detection system 100 to communicate data associated with at least one of the smoke detection system 100 and a protected area, from one or more detector modules 200-206, to a display module 300. In general, the smoke detection system 100 may include one or more detector modules 200-206, and at least one display module 300. The number of detector modules 200-206 may correspond to a number of channels selected for the system 100. The detector modules 200-206 may be configured to receive air from a protected area and detect smoke and/or compounds indicating smoke and/or fire in a protected area. The detector modules 200-206 are electrically and physically coupled to one or more backplanes, such as backplanes 104-112, with the number of backplanes being at least one more than the number of detector modules 200-206. When coupled to the backplanes 104-112, the detector modules 200-206 are configured to transmit power, signals, or both between detector modules.

The smoke detection system 100 further includes at least one display module 300 having a display window 302. The display module 300 may be electrically and physically coupled to the backplane 104-112, and configured to receive data signals associated with at least one of the smoke detection system 100 and the protected area, from the one or more detector modules 200-206. By way of example, data or data parameters (see, FIGS. 5C, 7A) from one or more detector modules 200-206 may include information associated with the smoke detection system 100 and/or the protected area, such as alarms, alerts, pre-alarms (e.g., active/triggered), temperature, airflow throughout the smoke detection network 400, date and/or time (and/or ranges), faults, warnings, conditions, inputs and outputs.

The method may also include in step 804 encoding the data received from one or more detector modules 200-206 with the display module 300. In step 806, the display module 300 may include a visual code generator module 310 configured to generate a visual code 304. The visual code 304 may include a two dimensional barcode or QR code, or a three dimensional visual code such as a hologram. In step 808, the display module 300 displays the generated visual code 304 in the display window 302 of the display module 300 where it may be captured and decoded by a mobile device 500. By way of example, a user may desire to obtain information about the smoke detection network 400. In this example, a user may interact with a display module 300 in cluster 402 through one or more display module menus (e.g., FIGS. 5A-5C, 7A) to generate a visual code 304. In another non-limiting example, the display module 300 may automatically generate a visual code 304, when the display module is configured with a learning mechanism.

In step 810, a mobile device 500 may decode the displayed visual code in wireless communication with the display module 300, 304, using for example, a mobile application 572. The mobile application 572 may be configured for example, to capture and decode the visual code 304. In the next step 812, the mobile device 500 may be configured to display the decoded data on the mobile device 500, in a user-readable form. A user-readable form may include at least one of text, an image, a portable document format, table, a graph, a chart, and a diagram.

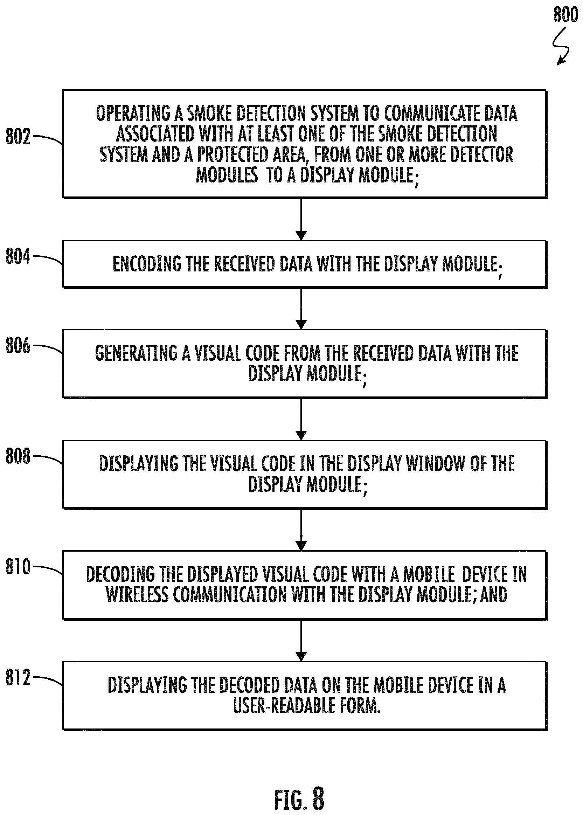

While the above description has described the method for monitoring conditions associated with a smoke detection system in FIG. 8 in a particular order, it should be appreciated that unless otherwise specifically required in the attached claims that the ordering of the steps may be varied.

As described above, embodiments can be in the form of processor-implemented processes and devices for practicing those processes, such as processor. Embodiments can also be in the form of computer program code containing instructions embodied in tangible media, such as floppy diskettes, CD ROMs, hard drives, or any other computer-readable storage medium, wherein, when the computer program code is loaded into and executed by a computer, the computer becomes a device for practicing the embodiments. Embodiments can also be in the form of computer program code, for example, whether stored in a storage medium, loaded into and/or executed by a computer, or transmitted over some transmission medium, loaded into and/or executed by a computer, or transmitted over some transmission medium, such as over electrical wiring or cabling, through fiber optics, or via electromagnetic radiation, wherein, when the computer program code is loaded into an executed by a computer, the computer becomes an device for practicing the exemplary embodiments. When implemented on a general-purpose microprocessor, the computer program code segments configure the microprocessor to create specific logic circuits.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present invention, which is defined by the claims. In addition, many modifications may be made to adapt a particular situation or material to the teachings of the present disclosure. Therefore, it is intended that the present invention not be limited to the particular embodiment disclosed as the best mode contemplated for carrying out this present invention , but that the present invention will include all embodiments falling within the scope of the claims.

## Claims

1. A system for monitoring conditions associated with a smoke detection system, comprising:
a first detector module comprising a sensor configured to detect smoke in a first volume of air received from a protected area, the first detector module electrically and physically coupled to a first backplane;
a second detector module comprising a sensor configured to detect smoke in a second volume of air received from the protected area, the second detector module electrically and physically coupled to a second backplane and to the first backplane so as to transmit power, signals, or both between the first and second detector modules;
a display module comprising a display window, the display module electrically and physically coupled to a third backplane, and to the first and second backplanes so as to transmit power, signals, or both between the display module and the first and second detector modules, the display module configured to:
receive data from the first and second detector modules associated with at least one of the smoke detection system and the protected area;
encode the received data;
generate a visual code from the received data; and
display the visual code in the display window; and
a mobile device in wireless communication with the display module, configured to capture and decode the visual code, and display the decoded data on the mobile device in a user-readable form.

2. The system of claim 1, wherein the visual code is two dimensional.

3. The system of claim 2, wherein the two dimensional visual code comprises a quick response code.

4. The system of claim 1, wherein the visual code is three dimensional.

5. The system of claim 4, wherein the three dimensional visual code comprises a hologram.

6. The system of any preceding claim, wherein the mobile device comprises at least one of a mobile phone, a personal digital assistant, a tablet, and a computer.

7. The system of any preceding claim, wherein the user-readable form comprises at least one of text, an image, a portable document format, a table, a graph, a chart, a diagram.

8. A method for monitoring conditions associated with a smoke detection system, comprising:
operating a smoke detection system to communicate data associated with at least one of the smoke detection system and a protected area, from one or more detector modules, to a display module;
encoding the received data with the display module;
generating a visual code from the received data with the display module;
displaying the visual code in the display window of the display module;
decoding the displayed visual code with a mobile device in wireless communication with the display module; and
displaying the decoded data on the mobile device in a user-readable form.

9. The method of claim 8, wherein the visual code is two dimensional.

10. The method of claim 9, wherein the two dimensional visual code comprises a quick response code.

11. The method of claim 8, wherein the visual code is three dimensional.

12. The method of claim 11, wherein the three dimensional visual code comprises a hologram.

13. The method of any of claims 8 to 12, wherein the user-readable form comprises at least one of text, an image, a portable document format, a table, a graph, a chart, a diagram.

14. The method of any of claims 8 to 13, comprising selecting data for encoding from one or more display module menus.

15. The method of any of claims 8 to 14, wherein the mobile device comprises at least one of a mobile phone, a personal digital assistant, a tablet, and a computer.
